Europäisches Patentamt

European Patent Office

Office européen des brevets

(11) Numéro de publication : **0 610 118 A1**

(19)

# (12) DEMANDE DE BREVET EUROPEEN

(21) Numéro de dépôt : **94400186.6**

(22) Date de dépôt : **28.01.94**

(51) Int. Cl.⁵ : **G01N 33/28,** G01N 25/52

(30) Priorité : **01.02.93 FR 9301038**

(43) Date de publication de la demande :
**10.08.94 Bulletin 94/32**

(84) Etats contractants désignés :
**AT BE CH DE DK ES FR GB GR IE IT LI LU NL
PT SE**

(71) Demandeur : **ELF ANTAR FRANCE
Tour Elf
2, Place de la Coupole
La Défense 6
F-92400 Courbevoie (FR)**

(72) Inventeur : **Cellier, Joseph
5, rue Colonel Manhès
F-69200 Venissieux (CH)**
Inventeur : **Mille, Guy
Saint Martin
F-69700 Echalas (FR)**
Inventeur : **Mottas, Alex
Vy d'Etraz La
CH-2014 Bole (CH)**

(74) Mandataire : **Dubois-Chabert, Guy et al
Société de Protection des Inventions
25, rue de Ponthieu
F-75008 Paris (FR)**

(54) **Procédé de mesure de l'indice de cétane de carburants d'alimentation des moteurs diesels et dispositif pour la mise en oeuvre de ce procédé.**

(57)    L'invention concerne l'étude, le contrôle et la fabrication de carburants pour moteurs diesels.

Selon l'invention, on mesure automatiquement l'indice de cétane d'un carburant d'alimentation d'un moteur diesel à partir de la valeur de l'indice de cétane d'au moins un carburant de référence et à partir des valeurs respectivement mesurées des délais d'auto-inflammation du carburant de référence et du carburant d'alimentation. Le moteur diesel fonctionne à vitesse constante et à rapport volumétrique de compression constant.

L'invention trouve son application dans les laboratoires de contrôle et de recherche ainsi que dans les unités de fabrication de carburants pour moteurs diesels, dans les établissements de traitement du pétrole brut.

EP 0 610 118 A1

**Domaine technique**

La présente invention concerne un procédé de mesure de l'indice de cétane de carburants d'alimentation de moteurs diesels, ainsi qu'un dispositif pour la mise en oeuvre de ce procédé.

Elle trouve son application dans les laboratoires de recherche, les laboratoires de contrôle et les unités de fabrication de ces carburants dans les établissements de traitement du pétrole brut.

**Etat de la technique antérieure**

L'indice de cétane d'un carburant pour moteur diesel est représentatif de sa qualité d'inflammabilité. Par convention, les indices de cétane, de l'heptaméthylnonane et du cétane normal ont respectivement pour valeur 15 et 100.

Une méthode de détermination de l'indice de cétane des carburants pour moteur diesel est décrite dans la norme ASTM D613.

Selon cette méthode, l'indice de cétane d'un carburant pour moteur diesel est déterminé par comparaison de sa qualité d'auto-inflammabilité avec celles de mélanges de carburants de référence d'indices de cétane connus.

Pour le carburant à analyser et pour chaque mélange de référence, on règle le rapport volumétrique de compression du moteur de manière à obtenir, pour le carburant à analyser et pour chacun des mélanges, un délai d'auto-inflammation prédéterminé (13°). On obtient ainsi les rapports volumétriques de compression des carburants de référence d'indices connus. Lorsque le rapport volumétrique de compression de carburant à analyser se situe entre les rapports volumétriques des mélanges de référence, on en déduit par interpolation l'indice de cétane de carburant à analyser. L'appareillage de mesure utilisé pour mettre en oeuvre cette méthode connue comprend essentiellement :

- un moteur monocylindre à rapport volumétrique de compression variable,
- un dispositif de mesure du délai d'auto-inflammation de carburant pouvant alimenter le moteur,
- une pompe à injection munie d'un moyen micrométrique qui permet de régler manuellement l'avance à l'injection,
- une disposition d'indication de l'avance à l'injection.

Cet appareillage doit fonctionner dans des conditions opératoires strictes précisées dans la norme ASTM D613.

Le réglage du rapport volumétrique de compression s'effectue au moyen d'un piston plongeur dont la position est modifiable par action manuelle sur un volant volumétrique.

De manière plus précise, pour déterminer l'indice de cétane d'un carburant d'alimentation du moteur, celui-ci est alimenté successivement par deux carburants de référence d'indices de cétane de valeurs connues puis par le carburant à analyser dont l'indice de cétane est à déterminer.

Conformément aux prescriptions de la norme ASTM D 613, l'avance à l'injection est réglée à la valeur 13° avant le point mort haut du piston du moteur. Pour chaque carburant, le rapport volumétrique de compression est réglé manuellement pour obtenir une valeur de délai d'auto-inflammation par rapport à l'instant d'injection, (exprimée en angle de la position du vilebrequin du moteur) égale à 13°, lue sur un indicateur de délai d'auto-inflammation.

A chaque opération de réglage du rapport volumétrique, la position du piston plongeur est notée.

A partir de ces résultats et des indices de cétane des carburant de référence, l'indice de cétane recherché est calculé par interpolation, comme indiqué plus haut.

Cette méthode est longue à mettre en oeuvre et nécessite de nombreuses opérations manuelles. Elle ne permet pas d'obtenir automatiquement la valeur de l'indice de cétane des carburants d'alimentation du moteur, par exemple sous forme de signaux électriques, représentatifs de ces indices.

Pour réguler la valeur d'indice de cétane d'un carburant en cours de fabrication, il est nécessaire de disposer de celle-ci de manière quasi continue. Ce qui est impossible à l'aide de la méthode décrite dans la norme ASTM D 613.

**Exposé de l'invention**

La présente invention a justement pour objet de remédier à cet inconvénient et notamment de fournir un procédé et un dispositif automatique de mesure d'indices de cétane des carburants pour moteurs diesels.

Ce procédé et ce dispositif sont utilisables aussi bien en laboratoire pour le contrôle d'échantillons de carburants, qu'en cours de fabrication d'un carburant pour la régulation de valeur de son indice de cétane, en intervenant sur la composition du mélange constituant ce carburant.

A cette fin, la présente invention propose un procédé de mesure de la valeur de l'indice de cétane d'un carburant d'alimentation de moteurs diesels, par comparaison avec une valeur connue d'un indice de cétane d'au moins un carburant de référence, ces carburants d'alimentation et de référence présentant respectivement des délais d'auto-inflammation, ce procédé consistant à utiliser un moteur diesel de mesure tournant à vitesse constante alimenté successivement par ledit carburant de référence et par ledit carburant d'alimentation, ledit moteur de mesure ayant un taux de compression constant et une avance constante à l'injection, caractérisé en ce que ce procédé consiste à calculer l'indice de cétane du carburant d'alimentation à partir de la valeur de l'indice de cétane du carburant de référence, et à partir des valeurs respectivement mesurées des délais d'auto-inflammation du carburant de référence et du carburant d'alimentation.

Selon une autre caractéristique, la présente invention propose un procédé caractérisé en ce que le moteur de mesure est alimenté successivement par deux carburants de référence d'indices respectifs de cétane connus puis par le carburant d'alimentation. La valeur de l'indice de cétane du carburant d'alimentation étant calculé par application de la formule suivante :

$$Icx = Ic1 + (Ic2 - Ic1)\frac{Dix - Di1}{Di2 - Di1}$$

dans laquelle :
. Icx est la valeur de l'indice de cétane du carburant d'alimentation,
. $Ic_1$ et $Ic_2$ sont respectivement les valeurs des indices de cétane des carburants de référence,
. $Di_1$ et $Di_2$ sont les valeur mesurées des délais respectifs d'auto-inflammation des carburants de référence,
. Dix est la valeur mesurée du délai d'auto-inflammation du carburant d'alimentation.

Les carburants de référence étant choisis pour que la valeur de l'indice de cétane du carburant d'alimentation soit comprise entre les valeurs respectives des indices de cétane des carburants de référence.

Selon une autre caractéristique, la présente invention propose un procédé caractérisé en ce que le moteur de mesure est alimenté au cours d'une phase d'étalonnage par un carburant de référence d'indice de cétane connu, puis périodiquement par un carburant d'alimentation en cours de fabrication, dont on veut connaître la valeur de l'indice de cétane. La valeur de l'indice de cétane du carburant d'alimentation étant calculée par application de la formule suivante :

$$Icx = Ic1 + K (Dix - Di1)$$

. Icx est la valeur de l'indice de cétane du carburant d'alimentation calculée périodiquement,
. Ic1 est la valeur connue de l'indice de cétane du carburant de référence,
. Di1 est la valeur mesurée au cours de la phase d'étalonnage du délai d'auto-inflammation du carburant de référence,
. Dix est la valeur mesurée périodiquement, du délai d'auto-inflammation du carburant d'alimentation
. K est une constante.

Selon une autre caractéristique, la présente invention propose un procédé caractérisé en ce que le moteur diesel ayant un cycle de fonctionnement prédéterminé, des valeurs d'indice de cétane du carburant d'alimentation sont calculées et mémorisées au cours d'un cycle de mesure correspondant à une pluralité de cycles de fonctionnement, une valeur moyenne de l'indice de cétane du carburant d'alimentation étant calculée pour ce cycle de mesure à partir des valeurs mémorisées de cet indice.

L'invention a aussi pour objet un dispositif de mesure de l'indice de cétane d'un carburant d'alimentation de moteurs diesels comportant :
- un moteur diesel de mesure tournant à vitesse constante, alimenté à débit constant, avec une avance à l'injection prédéterminée, et recevant successivement ledit carburant d'alimentation et au moins un carburant de référence ayant un indice de cétane connu, ces carburants d'alimentation et de référence présentant respectivement des délais d'auto-inflammation,
- des moyens pour alimenter successivement le moteur de mesure par le carburant de référence et par le carburant d'alimentation,
- un capteur de détection de chaque instant d'injection de carburant dans le moteur,
- un capteur de mesure de pression de chambre de combustion du moteur fournissant un signal de mesure de pression sur une sortie,
- des moyens de traitement du signal de mesure de pression pour ledit carburant de référence et ledit carburant d'alimentation et de traitement de la valeur de l'indice de cétane du ou des carburants de référence pour calculer la valeur de l'indice de cétane du carburant d'alimentation caractérisé en ce que le fait que les moyens de traitement comportent :
- un circuit de détection de pic de pression relié à la sortie du capteur de pression pour détecter dans le signal de sortie du capteur pression, l'instant d'apparition d'un pic de pression correspondant à une auto-inflammation pour le carburant de référence et pour le carburant d'alimentation, après l'instant d'injection,
- un circuit de mesure relié à une sortie du capteur d'injection et à une sortie du circuit de détection de pic de pression pour mesurer les délais séparant les instants d'injection et d'apparition de pics de pression, pour chacun desdits carburants de référence et d'alimentation, ces délais correspondant respectivement aux délais d'auto-inflammation desdits carburants,
- une unité de traitement reliée à une sortie du circuit de mesure et à des moyens de mémorisation pour calculer la valeur de l'indice de cétane du carburant d'alimentation à partir des indices de cétane du carburant de référence et des délais d'auto-inflammation des carburants de référence et d'alimentation.

Selon un premier mode de réalisation, l'invention a pour objet un dispositif caractérisé en ce que les moyens de mémorisation contiennent un programme de traitement, pour calculer l'indice de cétane du carburant d'alimentation par application de la formule suivante :

$$Icx = IC_1 + (IC_2 - IC_1)\frac{Dix - Di1}{Di2 - Di1}$$

dans laquelle :

. Icx est la valeur de l'indice de cétane du carburant d'alimentation,

. Ic1 et Ic2 sont respectivement les valeurs des indices de cétane des carburants de référence,

. Di1 et Di2 sont les valeurs mesurées des délais respectifs d'auto-inflammation des carburants de référence,

. Dix est la valeur mesurée du délai d'auto-inflammation du carburant d'alimentation.

Selon un deuxième mode de réalisation, l'invention propose un dispositif caractérisé en ce que les moyens de mémorisation contiennent un programme de traitement, pour calculer l'indice de cétane du carburant d'alimentation par application de la formule suivante :

$$Icx = Ic1 + K(Dix - Di1)$$

dans laquelle :

. Icx est la valeur de l'indice de cétane du carburant d'alimentation calculée périodiquement,

. Ic1 est la valeur connue de l'indice de cétane du carburant de référence,

. Di1 est la valeur mesurée au cours de la phase d'étalonnage du délai d'auto-inflammation du carburant de référence,

. Dix est la valeur mesurée périodiquement, du délai d'auto-inflammation du carburant d'alimentation

. K est une constante.

Selon une autre caractéristique du premier mode de réalisation, l'invention propose un dispositif caractérisé par le fait qu'il comporte en outre ;

- trois réservoirs pour stocker respectivement deux carburants de référence ayant des indices de cétane de valeurs prédéterminées connues et un carburant d'alimentation ayant un indice de cétane de valeur à mesurer, la valeur à mesurer étant comprise entre les valeurs prédéterminées,

- trois tuyaux de prélèvement reliés respectivement aux trois sorties des trois réservoirs et reliés à un tuyau commun d'alimentation du moteur,

- trois vannes à ouverture et fermeture commandables, interposées respectivement sur les trois tuyaux de prélèvement, ces vannes comprenant des entrées de commande reliées à des sorties de l'unité de traitement pour que le moteur soit alimenté successivement par les deux carburants de référence et par le carburant d'alimentation, pour mesurer les délais respectifs d'auto-inflammation des carburants de référence et du carburant d'alimentation pour calculer la valeur de l'indice de cétane du carburant d'alimentation.

Selon une autre caractéristique du deuxième mode de réalisation l'invention propose un dispositif caractérisé en ce que le fait qu'il compte en outre au moins deux réservoirs pour stocker respectivement deux carburants de base à mélanger pour constituer un carburant unique d'indice de cétane de valeur souhaitée :

- un réservoir pour stocker un carburant de référence ayant un indice de cétane connu ;

- un réservoir tampon pour stocker successivement le carburant de référence puis des échantillons du mélange de carburants de base ;

- un tuyau d'alimentation reliant le réservoir tampon avec l'alimentation du moteur ;

- deux tuyaux de vidange reliés respectivement à deux sorties des réservoirs de stockage des carburants de base et reliés à un tuyau collecteur commun dans lequel s'effectue le mélange ;

- un tuyau de prélèvement d'échantillons reliant ledit tuyau collecteur au réservoir tampon ;

- un tuyau de référence reliant la sortie du réservoir de stockage de carburant de référence à une sortie du réservoir tampon ;

- des vannes à ouverture et fermeture commandables, interposées respectivement sur les tuyaux de prélèvement d'échantillon et sur le tuyau de référence ;

- des vannes de régulation de débit des carburants de base interposées sur les tuyaux de vidange ;

- les vannes à ouverture et fermeture commandables comprenant des entrées de commande reliées à des sorties de l'unité de traitement pour commander le remplissage du réservoir tampon successivement par le carburant de référence puis par des échantillons du mélange, les vannes de régulation ayant des entrées de commande reliées respectivement à des sorties de moyens de régulation de débit, ces moyens de régulation étant reliés à une sortie de mesure de l'unité de traitement pour recevoir la valeur mesurée de l'indice de cétane du mélange, ces moyens de régulation recevant sur une autre entrée un signal de consigne correspondant à ladite valeur souhaitée de l'indice de cétane du mélange.

**Brève description des dessins**

L'invention sera mieux comprise à l'aide des dessins annexés dans lesquels :

- **la figure 1** représente schématiquement un premier mode de réalisation conforme au descriptif de l'invention utilisable en laboratoire pour la mesure de l'indice de cétane d'un carburant ;

- **la figure 2** représente schématiquement un deuxième mode de réalisation conforme au descriptif de l'invention utilisable pour la mesure en ligne et la régulation de l'indice de cétane d'un carburant en cours de fabrication ;
- **la figure 3** est un chronogramme des signaux essentiels intervenant dans les dispositifs des figures 1 et 2.

## Exposé détaillé de l'invention

D'une manière générale, le procédé et le dispositif de l'invention sont utilisés pour mesurer l'indice de cétane de carburants pour moteurs diesels. L'indice de cétane d'un carburant est caractéristique de son auto-inflammabilité, elle-même définie par le délai d'auto-inflammation du carburant. Ce délai est mesuré entre l'instant d'injection de la première goutte de carburant dans un moteur diesel et l'instant de l'auto-inflammation qui se manifeste après une forte et brusque montée de pression dans la chambre de combustion.

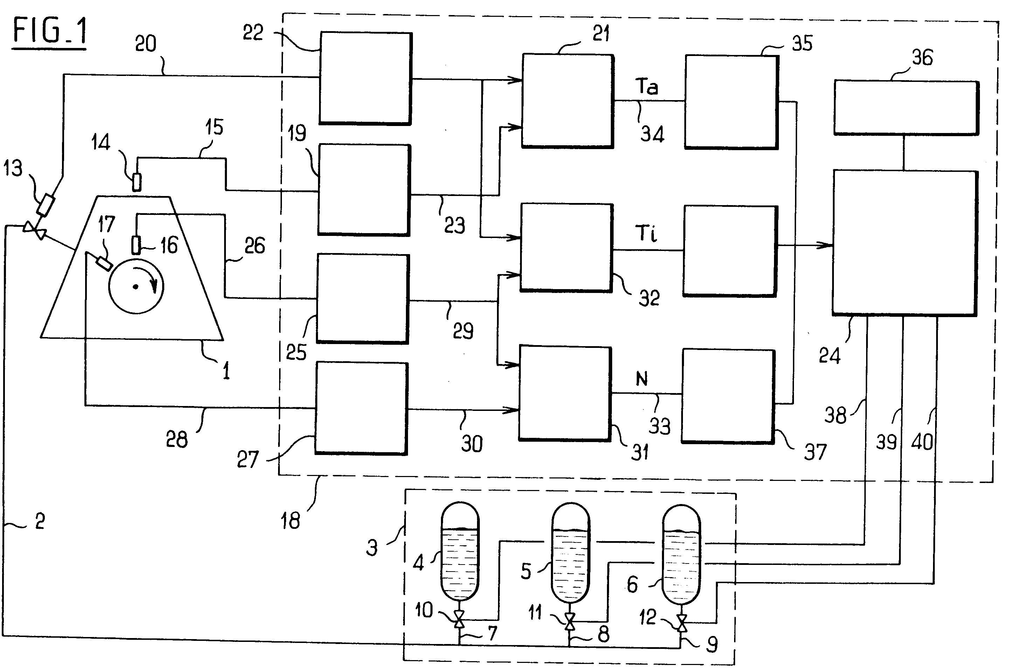

**La figure 1** représente schématiquement un premier mode de réalisation du dispositif de l'invention utilisable pour effectuer au laboratoire des mesures d'indice de cétane d'échantillons de carburants. La description de ce dispositif et de son fonctionnement permettront de mieux comprendre le procédé de l'invention.

Le dispositif, selon ce premier mode de réalisation, comporte un moteur diesel 1 de mesure alimenté en carburant à débit constant au moyen d'un tuyau 2 d'alimentation. Le débit peut être par exemple de 13ml/mn. Ce moteur tourne à une vitesse constante par exemple 900 tr/mn. L'avance à l'injection est réglée à une valeur fixe (par exemple 15° vilebrequin) par rapport au point mort haut du piston du moteur. La valeur mesurée de cette avance peut être affichée par un indicateur numérique de contrôle non représenté, placé à proximité du moteur .

Le rapport volumétrique de compression du moteur est défini comme le rapport entre les volumes respectifs de la chambre de combustion quand le piston est dans la position point mort bas puis dans la position point mort haut.

Selon le procédé de l'invention, ce rapport est fixé et il est réglé manuellement de façon connue au moyen d'un piston plongeur.

La vitesse, le débit de carburant d'alimentation, l'avance à l'injection et le rapport volumétrique de compression du moteur étant maintenus constants, chaque carburant d'alimentation présente un délai d'auto-inflammation représentatif de son auto-inflammabilité.

Le dispositif comporte des moyens 3 pour alimenter le moteur par au moins un carburant de référence et le carburant d'alimentation. Dans le premier mode de réalisation de l'invention ces moyens 3 comportent trois réservoirs 4,5 et 6 contenant respectivement le carburant à analyser et les deux carburants de référence d'indices de cétane connus de valeurs respectives Ic1 et Ic2. Ces réservoirs sont reliés à la pompe d'injection du moteur par des tuyaux appropriés 7,8,9 eux-mêmes reliés au tuyau d'alimentation 2 par des électrovannes 10,11,12. Le moteur est alimenté successivement par les trois carburants stockés dans les réservoirs, grâce aux actions d'ouverture et de fermeture d'électrovannes interposées respectivement sur les tuyaux 7,8,9.

Le dispositif comporte en outre divers capteurs :
- un capteur 13 de détection d'instant d'injection qui fournit une impulsion électrique à l'instant d'injection du carburant dans le moteur ;
- un capteur 14 de mesure de la pression dans la chambre de combustion qui fournit sur une sortie 15, un signal électrique représentatif de cette pression ;

Le dispositif peut aussi comporter :
- un capteur 16 de position, qui fournit un signal électrique correspondant au passage du piston par la position point mort haut ;
- un capteur 17 de position de référence qui fournit un signal électrique correspondant au passage du piston à la position choisie pour référence, avant la position point mort haut (par exemple 13°).

Le dispositif comporte aussi des moyens de traitement 18 du signal de mesure de pression fourni par la sortie 15 du capteur 14 pour les carburants de référence et le carburant d'alimentation et de traitement des valeurs de l'indice de cétane des carburants de référence. Ces moyens, comme on le verra plus loin en détail, permettent de calculer la valeur de l'indice de cétane du carburant d'alimentation fourni par le tuyau 2.

Les moyens de traitement 18 dans ce premier mode de réalisation comportent :
- un circuit 19 de détection de pic de pression relié à la sortie 15 du capteur 14 de pression pour détecter dans le signal de sortie de ce capteur, l'instant d'apparition d'un pic de pression. Ce pic correspond à l'auto-inflammation du carburant fourni au moteur, après l'instant d'injection. Cet instant d'injection est déterminé par le signal de sortie du capteur d'injection 13, ce signal est fourni par une sortie 20 de ce capteur 13 ;
- un circuit 21 de mesure, relié à la sortie 20 du capteur d'injection par exemple par l'intermédiaire d'un circuit 22 de mise en forme du signal fourni par la sortie 20 du capteur d'injection 13. Le circuit de mesure est aussi relié à une sortie 23 du circuit de détection 19 qui fournit un signal représentatif de l'instant d'apparition du pic de pression dans la chambre de combustion pour chaque carburant. Le circuit 21 permet de mesurer le délai séparant les instants d'injection et d'apparition de pics de pression, pour les carburants de référence et le carburant d'alimentation fournis au moteur. Ces délais correspondent respectivement aux délais d'auto inflammation de ces carburants;;

- une unité de traitement 24 reliée à la sortie 34 du circuit de mesure 21 du délai d'auto-inflammation par l'intermédiaire d'une mémoire tampon 35. Cette unité de traitement est aussi reliée à des moyens de mémorisation 36. Elle permet de calculer les valeurs des indices de cétane du carburant d'alimentation à partir des indices de cétane des carburants de référence et les valeurs des délais d'auto-inflammation des carburants de référence et d'alimentation.

Selon le premier mode de réalisation, les moyens de mémorisation 36 de l'unité de traitement 24 contiennent un programme de traitement, pour calculer l'indice de cétane du carburant d'alimentation par application de la formule suivante :

$$Icx = Ic1 + (Ic2 - Ic1)\frac{Dix - Di1}{Di2 - Di1}$$

dans laquelle :

. Icx est la valeur de l'indice de cétane du carburant d'alimentation,

. Ic1 et Ic2 sont respectivement les valeurs des indices de cétane des carburants de référence.

. Di1 et Di2 sont les valeurs mesurées des délais respectifs d'auto-inflammation des carburants de référence.

. Dix est la valeur mesurée du délai d'auto-inflammation du carburant d'alimentation.

Sur la **figure 1** on a également représenté :

- un circuit 25 de mise en forme qui reçoit le signal 26 du capteur 16 de point mort haut et qui fournit sur une sortie 29 un signal électrique impulsionnel représentatif de l'instant de passage du piston au point mort ;

- un circuit 27 de mise en forme qui reçoit le signal 28 du capteur 17 de référence et qui fournit sur une sortie 30 un signal électrique impulsionnel représentatif de l'instant de passage du piston à la position de référence (par exemple 13°) ;

- un circuit 31 de mesure de la vitesse, relié aux sorties 29 et 30, qui calcule la vitesse du moteur et fournit un signal 33 de mesure de cette vitesse ;

- un circuit 32 de mesure du délai d'injection relié à la sortie 29 de point mort haut et à la sortie 20 d'instant d'injection. Ce circuit de mesure calcule le délai d'avance à l'injection et fournit sur une sortie 34 un signal de mesure de ce délai.

Selon le premier mode de réalisation de l'invention, le dispositif comporte en outre un système d'échantillonnage 3 comprenant les éléments suivants :

- deux réservoirs 4,5 contenant respectivement deux carburants de référence d'indices de cétane connus de valeurs Ic1 et Ic2. Ces carburants sont choisis de manière à ce que leurs valeurs d'indice de cétane encadrent la valeur de l'indice de cétane du carburant à analyser ;

- un réservoir 6 contenant le carburant à analyser ;

- trois tuyaux de prélèvement 7,8,9 reliés respectivement aux trois sorties des trois réservoirs 4,5,6 et reliés à un tuyau 2 commun d'alimentation du moteur ;

- trois électrovannes 10,11,12 à ouverture et fermeture commandables, interposées respectivement sur les trois tuyaux de prélèvement, ces électrovannes comprenant des entrées de commande reliées à des sorties 38,39,40 de l'unité de traitement.

Le programme de l'unité de traitement 24, stocké dans les moyens de mémorisation 36 se déroule de manière que le moteur 1 soit alimenté successivement par les deux carburants de référence et par le carburant à analyser pendant des durées prédéterminées, par exemple 120 secondes chacun.

A chaque cycle moteur, le circuit 21 de mesure du délai d'auto-inflammation calcule ce délai dont la valeur est alors enregistrée dans la mémoire tampon 35. On obtient ainsi une pluralité de valeurs des délais d'auto-inflammation des carburants de référence et du carburant à analyser, qui sont traitées par l'unité de traitement 24 selon la formule indiquée ci-dessus.

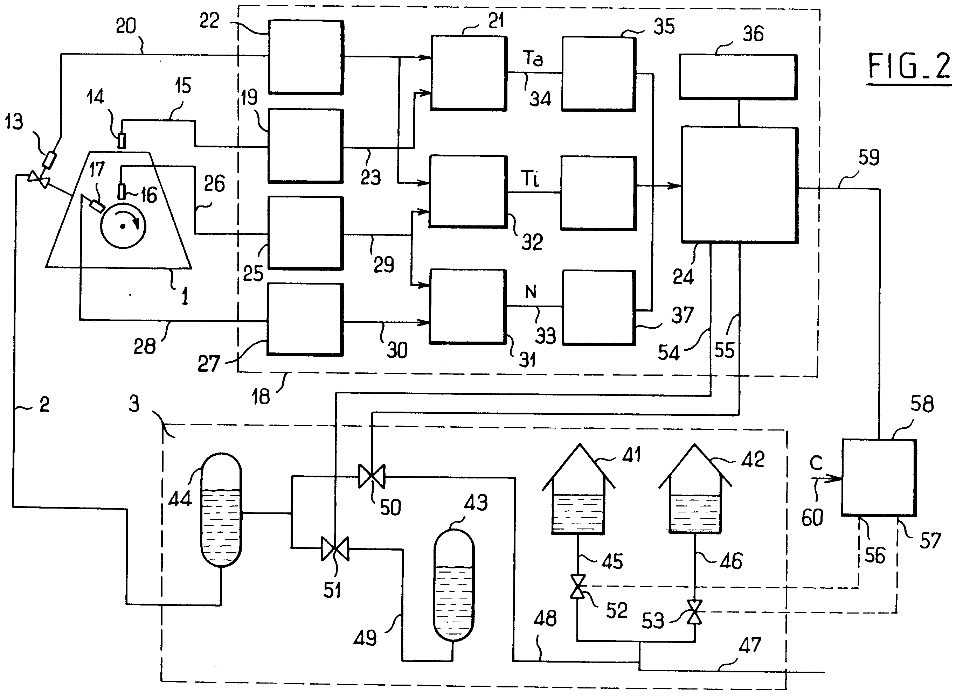

**La figure 2** représente schématiquement un deuxième mode de réalisation du dispositif de l'invention, utilisable pour effectuer en continu la mesure de la valeur d'indice de cétane d'un carburant en cours de fabrication. Cette mesure étant ensuite utilisée pour réguler la valeur d'indice de cétane du carburant fabriqué, a une valeur de consigne prédéterminée.

Le dispositif selon ce deuxième mode de réalisation comporte :

- un moteur diesel 1 de mesure équipé de capteurs de pression 14 et d'instant d'injection 13, tels que décrits pour le premier mode de réalisation fonctionnant dans les mêmes conditions ;

- des moyens de traitement 18 des signaux fournis par ces capteurs, tels que décrits pour le premier mode de réalisation. Comme dans le premier mode de réalisation, un programme est enregistré dans les moyens de mémorisation 36. Ce programme particulier à ce deuxième mode de réalisation, permet à l'unité de traitement d'effectuer des calculs et de fournir des commandes qui apparaîtront plus loin.

L'unité de traitement 24 comporte en outre une sortie 59 fournissant un signal analogique 59 représentatif de la valeur de l'indice de cétane du carburant en cours de fabrication.

Selon ce deuxième mode de réalisation, les moyens 3 d'alimentation du moteur sont constitués par les éléments suivants :

- au moins deux réservoirs 41,42 pour stocker deux carburants de base à mélanger pour constituer un carburant unique d'indice de cétane de valeur souhaitée ;
- un réservoir 43, pour stocker un carburant de référence ayant un indice de cétane de valeur connue.
- un réservoir tampon 44 pour stocker successivement un échantillon du carburant de référence en début d'opération puis des échantillons du mélange de carburants de base.
- un tuyau d'alimentation 2 reliant le réservoir tampon avec l'alimentation du moteur.
- Deux tuyaux 45,46 de vidange reliés respectivement à deux sorties des réservoirs 41,42 de stockage des carburants de base et reliés à un tuyau collecteur 47 commun dans lequel s'effectue le mélange souhaité.
- Un tuyau 48 de prélèvement d'échantillons reliant le tuyau collecteur 47 au réservoir tampon 44.
- Un tuyau de référence 49 reliant la sortie du réservoir de stockage du carburant de référence à une entrée du réservoir tampon.
- Des électrovannes 50,51 à ouverture et fermeture commandables, interposées respectivement sur les tuyaux de prélèvement d'échantillon 48 et sur le tuyau de référence 49.
- Des vannes 52,53 de régulation de débit des carburants de base interposées sur les tuyaux de vidange 45,46.

Les électrovannes 50,51 à ouverture et fermeture commandables comprenant des entrées de commande reliées à des sorties 54,55 de l'unité de traitement pour commander le remplissage du réservoir tampon, successivement par le carburant de référence puis par des échantillons du mélange. Les vannes de régulation 52,53 ont des entrées de commande reliées respectivement à des sorties 56,57 des moyens de régulation de débit 58, ces moyens de régulation sont reliés à une sortie de mesure 59 de l'unité de traitement pour recevoir la valeur mesurée de l'indice de cétane du mélange, ces moyens de régulation reçoivent sur une autre entrée 60 un signal de consigne correspondant à la valeur souhaitée de l'indice de cétane du mélange.

Selon ce deuxième mode de réalisation, au cours d'une première phase qui précède une fabrication de carburant, le réservoir tampon 44 est rempli de carburant de référence par ouverture de l'électrovanne 51, l'électrovanne 50 étant fermée. Ensuite le moteur 1 est alimenté pendant une durée prédéterminée, par exemple 120 secondes, au cours de laquelle le délai d'auto-inflammation du carburant de référence est déterminé par les moyens de traitement 18.

Cette opération d'étalonnage étant terminée, le réservoir tampon 44 est vidangé, puis rempli par un échantillon de carburant en cours de fabrication, prélevé dans le tuyau commun 17 par ouverture de l'électrovanne 50 et fermeture de l'électrovanne 51.

Le moteur étant alimenté par un échantillon du carburant en cours de fabrication pendant un temps prédéterminé, par exemple 120 secondes, les moyens de traitement 18 mesurent le délai d'auto-inflammation de cet échantillon de carburant et calculent la valeur de son indice d'octane par application de la formule suivante :

$$Icx = Ic1 + K(Dix - Di1)$$

dans laquelle :

. Icx est la valeur de l'indice de cétane du carburant en cours de fabrication ;

. Ic1 est la valeur connue de l'indice de cétane du carburant de référence ;

. Di1 est la valeur du délai d'auto-inflammation du carburant de référence mesurée au cours de la phase d'étalonnage ;

. Dix est la valeur mesurée du délai d'auto-inflammation du carburant en cours de fabrication;

. K est une constante.

Ensuite le réservoir tampon 44 est vidangé puis rempli par un nouvel échantillon du carburant en cours de fabrication et un nouveau cycle d'analyse se déroule.

L'opération est répétée jusqu'à la fin de fabrication de la quantité de carburant souhaité.

Le signal analogique 59 fournit par la sortie de l'unité de traitement 24, est représentatif. Cette valeur de l'indice de cétane du carburant en cours de fabrication, est comparée par les moyens de régulation 58 à la valeur souhaitée 60 pour l'indice de cétane du carburant fabriqué. Ces moyens de régulation 58 fournissent des signaux électriques sur les sorties 56 et 57 pour commander l'ouverture et la fermeture des vannes 52,53 de sorte que l'écart entre la valeur souhaitée et la valeur mesurée de l'indice de cétane soit nul.

Selon les deux modes de réalisation, l'unité de traitement dispose de moyens de visualisation non représentés sur les figures 1 et 2, qui permettent d'afficher la valeur de l'indice de cétane des carburants de référence et du carburant à analyser ainsi que d'autres paramètres. A titre d'exemple, on peut citer la vitesse de rotation du moteur, le temps d'analyse, les délais d'auto-inflammation des carburants de référence et d'alimentation, le délai d'avance à l'injection.

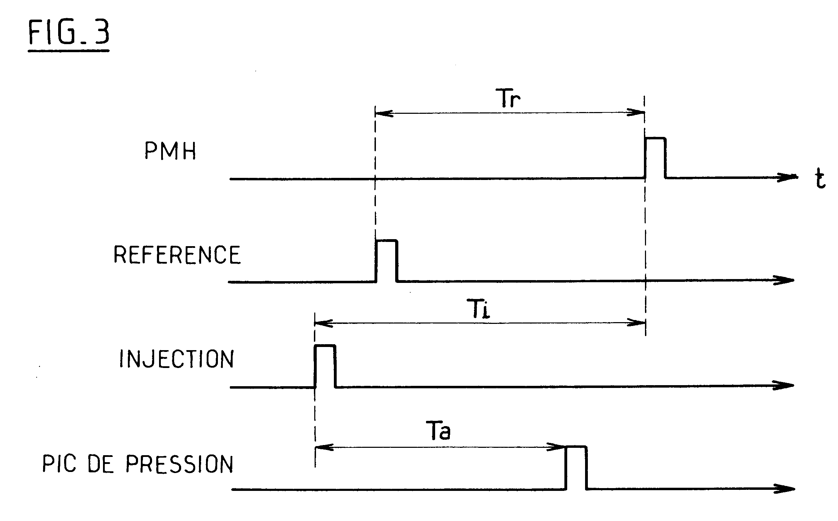

La figure 3 est un chronogramme des signaux essentiels intervenant dans les dispositifs représentés schématiquement par les figures 1 et 2.

L'intervalle de temps $T_i$ qui sépare le signal de début d'injection du signal de position de point mort haut (PMH) représente le délai d'avance à l'injection.

L'intervalle de temps $T_a$ qui sépare le début du signal d'injection du début du signal de pic de pression représente le délai d'auto-inflammation du carburant qui alimente le moteur.

L'intervalle de temps $T_r$ qui sépare l'impulsion de position de référence, de l'impulsion de position de point mort haut est constant quant le moteur tourne à vitesse constante. Cette valeur $T_r$ permet de calculer la vitesse de rotation du moteur.

## Revendications

1 - Procédé de mesure de la valeur de l'indice de cétane d'un carburant d'alimentation de moteurs diesels, par comparaison avec une valeur connue d'un indice de cétane d'au moins un carburant de référence, ces carburants d'alimentation et de référence présentant respectivement des délais d'auto-inflammation, ce procédé consistant à utiliser un moteur diesel de mesure tournant à vitesse constante alimenté successivement par ledit carburant de référence et par ledit carburant d'alimentation, ledit moteur de mesure ayant un taux de compression constant et une avance constante à l'injection, caractérisé en ce que ce procédé consiste à calculer l'indice de cétane du carburant d'alimentation à partir de la valeur de l'indice de cétane du carburant de référence, et à partir des valeurs respectivement mesurées des délais d'auto-inflammation du carburant de référence et du carburant d'alimentation.

2 - Procédé selon la revendication 1, caractérisé en ce que le moteur de mesure est alimenté successivement par deux carburants de référence d'indices respectifs de cétane connus puis par le carburant d'alimentation. La valeur de l'indice de cétane du carburant d'alimentation étant calculée par application de la formule suivante :

$$Icx = Ic1 + (Ic2 - Ic1)\frac{Dix - Di1}{Di2 - Di1}$$

dans laquelle :

Icx est la valeur de l'indice de cétane du carburant d'alimentation,

Ic1 et Ic2 sont respectivement les valeurs des indices de cétane des carburants de référence,

Di1 et Di2 sont les valeurs mesurées des délais respectifs d'auto-inflammation des carburants de référence,

Dix est la valeur mesurée du délai d'auto-inflammation du carburant d'alimentation.

Les carburants de référence étant choisis pour que la valeur de l'indice de cétane du carburant d'alimentation soit comprise entre les valeurs respectives des indices de cétane des carburants de référence.

3 - Procédé selon la revendication 1 caractérisé en ce que le moteur de mesure est alimenté au cours d'une phase d'étalonnage par un carburant de référence d'indice de cétane connu, puis périodiquement par un carburant d'alimentation en cours de fabrication, dont on veut connaître la valeur de l'indice de cétane. La valeur de l'indice de cétane du carburant d'alimentation étant calculée par application de la formule suivante :

$$Icx = Ic1 + K (Dix - Di1)$$

Icx est la valeur de l'indice de cétane du carburant d'alimentation calculée périodiquement,

Ic1 est la valeur connue de l'indice de cétane du carburant de référence,

Di1 est la valeur mesurée au cours de la phase d'étalonnage du délai d'auto-inflammation du carburant de référence,

Dix est la valeur mesurée périodiquement,

K est une constante.

4 - Procédé selon l'une quelconque des revendications 2 et 3, caractérisé en ce que le moteur diesel ayant un cycle de fonctionnement prédéterminé, des valeurs d'indice de cétane du carburant d'alimentation sont calculées et mémorisées au cours d'un cycle de mesure correspondant à une pluralité de cycles de fonctionnement, une valeur moyenne de l'indice de cétane du carburant d'alimentation étant calculée pour ce cycle de mesure à partir des valeurs mémorisées de cet indice.

5 - Dispositif de mesure de l'indice de cétane d'un carburant d'alimentation de moteurs diesels comportant:

- un moteur diesel de mesure tournant à vitesse constante, alimenté à débit constant, avec une avance à l'injection prédéterminée, et recevant successivement ledit carburant d'alimentation et au moins un carburant de référence ayant un indice de cétane connu, ces carburants d'alimentation et de référence présentant respectivement des délais d'auto-inflammation ;
- des moyens pour alimenter successivement le moteur de mesure par le carburant de référence et par le carburant d'alimentation ;
- un capteur de détection de chaque instant d'injection de carburant dans le moteur ;
- un capteur de mesure de pression de chambre de combustion du moteur fournissant un signal de mesure de pression sur une sortie ;
- des moyens de traitement du signal de mesure de pression pour ledit carburant de référence et ledit carburant d'alimentation et de traitement de la valeur de l'indice de cétane du ou des carburants de référence pour calculer la valeur de l'indice de cétane du carburant d'alimentation caractérisé par le fait que les moyens de traitement comportent ;
- un circuit de détection de pic de pression relié à la sortie du capteur de pression pour détecter dans le signal de sortie du capteur pression, l'instant d'apparition d'un pic de pression correspondant à une auto-inflammation pour le carburant de référence et pour le carburant d'alimentation, après l'instant d'injection ;

- un circuit de mesure relié à une sortie du capteur d'injection et à une sortie du circuit de détection de pic de pression pour mesurer les délais séparant les instants d'injection et d'apparition de pics de pression, pour chacun desdits carburants de référence et d'alimentation, ces délais correspondant respectivement aux délais d'auto-inflammation desdits carburants ;

- une unité de traitement reliée à une sortie du circuit de mesure et à des moyens de mémorisation pour calculer la valeur de l'indice de cétane du carburant d'alimentation à partir des indices de cétane du carburant de référence et des délais d'auto-inflammation des carburants de référence et d'alimentation.

**6** - Dispositif selon la revendication 5 caractérisé en ce que les moyens de mémorisation contiennent un programme de traitement, pour calculer l'indice de cétane du carburant d'alimentation par application de la formule suivante :

$$Icx = Ic1 + (Ic2 - Ic1)\frac{Dix - Di1}{Di2 - Di1}$$

dans laquelle :
Icx est la valeur de l'indice de cétane du carburant d'alimentation,
Ic1 et Ic2 sont respectivement les valeurs des indices de cétane des carburants de référence,
Di1 et Di2 sont les valeurs mesurées des délais respectifs d'auto-inflammation des carburants de référence,
Dix est la valeur mesurée du délai d'auto-inflammation du carburant d'alimentation.

**7** - Dispositif selon la revendication 5 caractérisé en ce que les moyens de mémorisation contiennent un programme de traitement, pour calculer l'indice de cétane du carburant d'alimentation par application de la formule suivante :

$$Icx = Ic1 + K (Dix - Di1)$$

dans laquelle :
Icx est la valeur de l'indice de cétane du carburant d'alimentation calculée périodiquement,
Ic1 est la valeur connue de l'indice de cétane du carburant de référence,
Di1 est la valeur mesurée au cours de la phase d'étalonnage du délai d'auto-inflammation du carburant de référence,
Dix est la valeur mesurée périodiquement,
K est une constante.

**8** - Dispositif selon la revendication 6 caractérisé par le fait qu'il comporte en outre :

- trois réservoirs pour stocker respectivement deux carburants de référence ayant des indices de cétane de valeurs prédéterminées connues et un carburant d'alimentation ayant un indice de cétane de valeur à mesurer, la valeur à mesurer étant comprise entre les valeurs prédéterminées ;

- trois tuyaux de prélèvement reliés respectivement aux trois sorties des trois réservoirs et reliés à un tuyau commun d'alimentation du moteur ;

- trois vannes à ouverture et fermeture commandables interposées respectivement sur les trois tuyaux de prélèvement, ces vannes comprenant des entrées de commande reliées à des sorties de l'unité de traitement pour que le moteur soit alimenté successivement par les deux carburants de référence et par le carburant d'alimentation, pour mesurer les délais respectifs d'auto-inflammation des carburants de référence et du carburant d'alimentation pour calculer la valeur de l'indice de cétane du carburant d'alimentation.

**9** - Dispositif selon la revendication 7 caractérisé par le fait qu'il compte en outre au moins deux réservoirs pour stocker respectivement deux carburants de base à mélanger pour constituer un carburant unique d'indice de cétane de valeur souhaitée;

- un réservoir pour stocker un carburant de référence ayant un indice de cétane connu ;

- un réservoir tampon pour stocker successivement le carburant de référence puis des échantillons du mélange de carburants de bases ;

- un tuyau d'alimentation reliant le réservoir tampon avec l'alimentation du moteur ;

- deux tuyaux de vidange reliés respectivement à deux sorties des réservoirs de stockage des carburants de base et reliés à un tuyau collecteur commun dans lequel s'effectue le mélange.

- un tuyau de prélèvement d'échantillons reliant ledit tuyau collecteur au réservoir tampon ;

- un tuyau de référence reliant la sortie du réservoir de stockage de carburant de référence à une entrée du réservoir de tampon ;

- des vannes à ouverture et fermeture commandables, interposées respectivement sur les tuyaux de prélèvement d'échantillon et sur le tuyau de référence ;

- des vannes de régulation de débit des carburants de base interposées sur les tuyaux de vidange ;

- les vannes à ouverture et fermeture commandables comprenant des entrées de commande reliées à des sorties de l'unité de traitement pour commander le remplissage du réservoir tampon successivement par le carburant de référence puis par des échantillons du mélange, les vannes de régulation ayant des entrées de commande reliées respectivement à des sorties de moyens de régulation de débit, ces moyens de régulation étant reliés à une sortie de mesure de l'unité de traitement pour recevoir la valeur mesurée de l'indice de cétane du mélange, ces moyens de régulation recevant sur une autre entrée un signal de consigne correspondant à ladite valeur souhaitée de l'indice de cétane du mélange.

FIG.1

EP 0 610 118 A1

FIG. 2

FIG.3

EP 0 610 118 A1

Office européen
des brevets

**RAPPORT DE RECHERCHE EUROPEENNE**

Numero de la demande

EP 94 40 0186

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.5) |
|---|---|---|---|
| X | DE-A-12 70 838 (MOBIL OIL CORPORATION) 20 Juin 1968 | 1,4,5 | G01N33/28 G01N25/52 |
| A | * colonne 1, ligne 1 - colonne 12, ligne 20; figures * * colonne 16, ligne 5 - ligne 56; revendications * * colonne 17, ligne 48 - colonne 21, ligne 15 * | 2,3,6-9 | |
| | --- | | |
| X | DATABASE WPI Week 8908, Derwent Publications Ltd., London, GB; AN 89-059996 & SU-A-1 416 910 (V.M.SHAPRANOV) 15 Août 1988 * abrégé * | 1 | |
| | --- | | |
| A | US-A-3 520 173 (W.C.HADLEY) * le document en entier * | 1-9 | |
| | --- | | |
| A | EP-A-0 143 571 (MOBIL OIL CORPORATION) * le document en entier * | 1-9 | DOMAINES TECHNIQUES RECHERCHES (Int.Cl.5) |
| | --- | | G01N |
| A | DATABASE WPI Week 9144, Derwent Publications Ltd., London, GB; AN 91322778 & SU-A-1 608 581 (CHEMOTOLOGY RES INS) 23 Novembre 1990 * abrégé * | 1,5 | |
| | ----- | | |

Le présent rapport a été établi pour toutes les revendications

| Lieu de la recherche | Date d'achèvement de la recherche | Examinateur |
|---|---|---|
| LA HAYE | 24 Mai 1994 | Brock, T |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
.........................................................................
& : membre de la même famille, document correspondant

EPO FORM 1503 03.82 (P04C02)